(19)

Europäisches
Patentamt
European
Patent Office
Office européen
des brevets

(11) **EP 4 030 439 A1**

(12) **EUROPEAN PATENT APPLICATION**

(43) Date of publication:
**20.07.2022 Bulletin 2022/29**

(21) Application number: **21151287.6**

(22) Date of filing: **13.01.2021**

(51) International Patent Classification (IPC):
***G16H 20/10*** *(2018.01)*

(52) Cooperative Patent Classification (CPC):
**G16H 70/40; G16H 10/40; G16H 20/10;
G16H 50/20**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **DELLIMORE, Kiran Hamilton J.
5656 AE Eindhoven (NL)**
• **HUIJBREGTS, Laurentia Johanna**
**5656 AE Eindhoven (NL)**
• **JOHNSON, Mark Thomas**
**5656 AE Eindhoven (NL)**
• **GERHARDT, Lutz Christian**
**5656 AE Eindhoven (NL)**
• **BONOMI, Alberto Giovanni**
**5656 AE Eindhoven (NL)**
• **PELSSERS, Eduard Gerard Marie**
**5656 AE Eindhoven (NL)**

(74) Representative: **Philips Intellectual Property &
Standards
High Tech Campus 52
5656 AG Eindhoven (NL)**

(54) **DETERMINING A TIME WINDOW REGARDING THE INTAKE OF A SUBSTANCE BY A SUBJECT**

(57) According to an aspect, there is provided a method comprising: receiving (402), from a sensor, first data indicative of a concentration of a first substance in sweat secreted from sweat glands of a subject; determining (404) a relationship between (i) a time of occurrence of a characteristic event in relation to the concentration of the first substance in sweat and (ii) a time of occurrence of the characteristic event in relation to the concentration of the first substance in blood; and determining (406), based on the relationship and the received first data, a time window regarding the intake of a second substance by the subject. Another aspect provides an apparatus for carrying out this method.

Fig. 4

**Description**

FIELD OF THE INVENTION

The invention relates to determining a time window regarding the intake of a substance by a subject and, more particularly, to determining such a time window based on data acquired in respect of sweat of the subject.

BACKGROUND OF THE INVENTION

Administering the correct dose of medication to a subject (e.g. a human or an animal) at the correct time can be crucial, for example in the case of medication used for the treatment of Parkinson's disease or diabetes or for sedation and/or analgesia (i.e. pain relief) during medical procedure.

It is known that substances that are taken by or administered to a subject, such as medication, can be detected not only in the subject's blood, but also to some extent in the subject's sweat. However, a substance may be present in a subject's blood at a concentration different to (e.g. higher or lower than) the concentration at which the substance is present in the subject's sweat. This lack of correspondence may be due to the mechanisms by which substances are transported from blood via interstitial fluid to the sweat glands, potential production in the sweat glands, excretion by the sweat glands and possible reabsorption in the sweat ducts. Moreover, the rate at which a substance appears in (e.g. is detectable in) the subject's blood following intake of the substance may be quicker than the rate at which the substance appears in (e.g. is detectable in) the subject's sweat. It is also known that, after a one-time administration, over time, the concentration of a xenobiotic substance in blood and in sweat reduces. Thus, by simply measuring the concentration of the substance in the subject's sweat, it can be difficult to determine an accurate and up-to-date concentration of the substance in the subject's blood.

For example, when medication is taken by or administered to a subject (e.g. intravenously), the medication may be transported around the subject's body through the blood vessels and capillaries and into tissues surrounding the capillaries. For medication to have the desired effect, a certain amount of its active substance should be present in the bloodstream. After a first bolus or dose of a medication has been administered, the next bolus or dose should be delivered at the correct time in order to avoid an overdose and to prevent the therapeutic effect of the medication wearing off in the subject. However, it can be difficult to know when a second bolus or dose should be taken or administered.

SUMMARY OF THE INVENTION

Thus, there is a desire for a method of determining an appropriate time window regarding the intake of a substance by a subject based on measurements acquired in respect of the subject's sweat. The invention is defined by the independent claims. The dependent claims define advantageous embodiments.

Embodiments of the present disclosure provide a mechanism by which a measurement of a concentration of a substance in a subject's sweat can be used to determine a time window regarding the intake of another substance or of a further intake of the same substance. This is achieved by determining a relationship between the concentration of the substance in the subject's sweat and the concentration of the substance in the subject's blood. The inventors have recognized that, by determining a time difference between the occurrence of a particular event (e.g. a maximum) in relation to the concentration of the substance in the subject's sweat and the occurrence of a particular event (e.g. the same event) in relation to the concentration of the substance in the subject's blood, it is possible to determine a time window regarding the suitable (e.g. safe) or unsuitable (e.g. unsafe) delivery or intake of a further amount of a substance.

According to a first specific aspect, there is provided a method comprising: receiving, from a sensor, first data indicative of a concentration of a first substance in sweat secreted from sweat glands of a subject; determining a relationship between (i) a time of occurrence of a characteristic event in relation to the concentration of the first substance in sweat and (ii) a time of occurrence of the characteristic event in relation to the concentration of the first substance in blood; and determining, based on the relationship and the received first data, a time window regarding the intake of a second substance by the subject.

The method may further comprise determining, based on the relationship and the received first data, a concentration of the first substance in blood of the subject.

In some embodiments, the method may further comprise receiving, prior to receiving the first data, second data indicative of the time of occurrence of the characteristic event in relation to the concentration of the first substance in blood.

The method may comprise generating a signal to alert a user about the determined time window. In some embodiments, the method may comprise generating a control signal to control delivery of the second substance at a time based on the determined time window.

The method may, in some embodiments, further comprise obtaining physiological data relating to the subject. Determining the time window may be further based on the physiological data.

The method may further comprise obtaining at least one of a first pharmacological model relating to the first substance and a second pharmacological model relating to the second substance. Determining the time window may be further based on at least one of the first pharmacological model and the second pharmacological model.

Determining the relationship may be based on at least one of: a determination of the time taken for the first substance to be detected in blood of the subject following intake of the first substance by the subject; an estimate of the time taken for the first substance to be detected in blood of the subject following intake of the first substance the subject, the estimate based on a population of people; an indication of the time taken for an effect of the first substance to be detected by a user following intake of the first substance by the subject; and an indication of the time taken for an effect of the first substance to be detected by a sensor following intake of the first substance by the subject.

In some embodiments, the received first data may comprise data indicative of a concentration of a metabolite of the first substance in the secreted sweat from sweat glands of a subject over a period of time. Determining the relationship may comprise determining a relationship between the time of occurrence of a characteristic event in relation to the concentration of the metabolite of the first substance in sweat and the time of occurrence of the characteristic event in relation to the concentration of the metabolite of the first substance in blood.

The method may further comprise determining, using a pharmacological model relating to the first substance and physiological data relating to the subject, a metabolic rate of the first substance in the subject. Determining the time window may be further based on the determined metabolic rate.

In some embodiments, the first substance and the second substance may interact with one another. In some embodiments, a metabolic product of the first substance and the second substance may interact with one another.

According to a second specific aspect, there is provided a computer program product comprising a non-transitory computer-readable medium, the computer-readable medium having computer-readable code embodied therein, the computer-readable code being configured such that, on execution by a suitable computer or processor, the computer or processor is caused to perform the method of any of the preceding claims.

According to a third specific aspect, there is provided an apparatus comprising a processor configured to perform the steps of the methods disclosed herein.

According to a fourth specific aspect, there is provided a system comprising: a sweat sensor for measuring data indicative of a characteristic of sweat generated by sweat glands of a subject; and an apparatus as disclosed herein. The first data may be received from the sweat sensor.

The system may further comprise a user interface configured to provide a notification to the subject at a time based on the determined time window.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1 is a graph showing an example of a concentration of a substance in blood for two consecutive intakes;
Fig. 2 is a graph showing a further example of a concentration of a substance in blood for two consecutive intakes;
Fig. 3 is a graph showing a further example of a concentration of a substance in blood for two consecutive intakes;
Fig. 4 is a flowchart of an example of a method according to various embodiments of the disclosure;
Fig. 5 is a graph showing an example of the change in concentration of a substance in blood and sweat as a function of time;
Fig. 6 is a graph showing a further example of the change in concentration of a substance in blood and sweat as a function of time;
Fig. 7 is the graph of Fig. 6 with various time regions indicated;
Fig. 8 is a flowchart of a further example of a method according to various embodiments of the disclosure;
Fig. 9 is a graph showing a further example of the change in concentration of a substance in blood and sweat as a function of time;
Fig. 10 is a graph showing a further example of the change in concentration of a substance in blood and sweat as a function of time;
Fig. 11 is a graph showing a further example of the change in concentration of a substance in blood and sweat as a function of time;
Fig. 12 is a schematic illustration of an example of a processor in communication with a computer-readable medium;
Fig. 13 is a schematic illustration of an example of an apparatus in accordance with various embodiments of the disclosure; and
Fig. 14 is a schematic illustration of an example of a system according to various embodiments of the disclosure.

DETAILED DESCRIPTION OF EMBODIMENTS

Embodiments disclosed herein provide a mechanism by which a time window relating to the safe intake or administration of a substance can be determined, based on a measurement of the concentration of a previously-taken substance in a subject's sweat, and knowledge of a relationship between the concentration of the previously-taken substance in the subject's sweat and the concentration of the previously-taken substance in the subject's blood. The substances referred to herein may comprise medication (e.g. drugs), ingredients (e.g. of food or drink) or any other substance that may be consumed by, ingested by, taken by or administered to a subject (e.g. a person) or a product of such a substance once the substance has been metabolized by the subject's body. As will become apparent, the invention is particularly advantageous in the field of clinical care, where a subject is to take or be administered medication. In such an example, the subject may take a first dose of a first medication, and may need to take a second dose of the same medication, or a dose of a second medication, at some later time. As medication is absorbed into tissues of the subject, the concentration of the medication reduces over time, and it may be desirable (e.g. particularly if the medication is being taken for pain relief purposes) that the second dose is taken before the concentration of the medication from the first dose reduces below a threshold at which the medication is therapeutically effective. Furthermore, it is important that the second dose is not taken too early, while the concentration of the medication from the first dose is still relatively high, because this could result in an overdose.

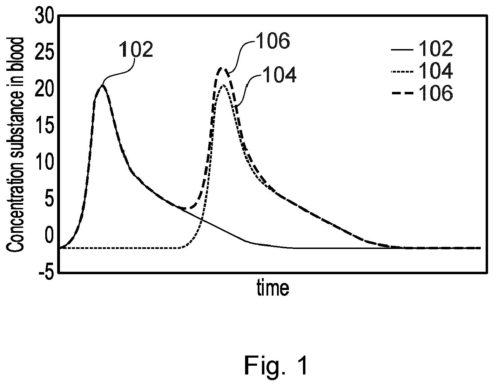

Figs. 1, 2 and 3 are graphs showing examples of the resulting concentration (in arbitrary units) of a substance in a subject's blood when first and second doses of the substance are taken at different times relative to one another. In the graphs are shown in Fig. 1, 2 and 3, a line 102 represents a concentration of a first dose of the substance in the subject's blood, a line 104 represents a concentration of a second dose of the substance in the subject's blood and a line 106 represents a combination (e.g. a sum) of the total concentration of the substance in the subject's blood resulting from the first dose of the substance and the second dose of the substance. In the examples shown, it is assumed that the total concentration should not rise above 25 arbitrary units (a.u.) and should not fall below 5 a.u. This relates to an example where a concentration exceeding 25 a.u. could be harmful (e.g. toxic) to the subject and a concentration lower than 5 a.u. is insufficient to have the desired therapeutic effect.

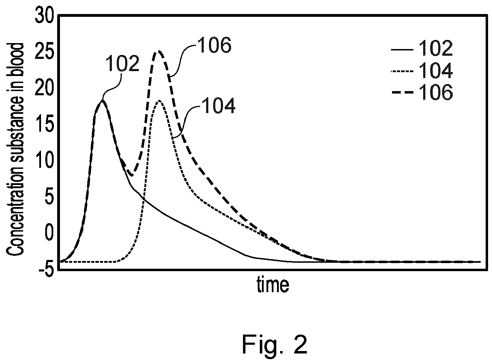

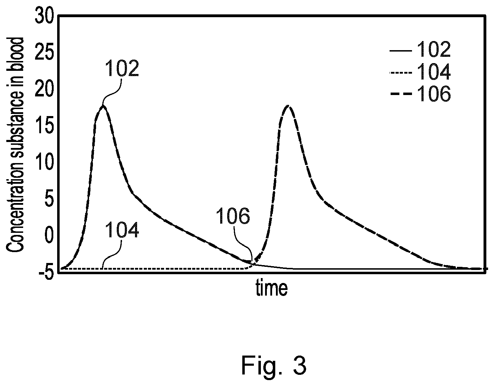

In Fig. 1, the second dose (line 104) is taken at an appropriate time after the first dose (line 102) is taken, such that the total concentration of the substance (line 106) does not drop below a level at which the medication becomes ineffective, and does not increase beyond a level which could be dangerous to the subject. The graph shown in Fig. 2 represents a scenario where the second dose (line 104) is taken too soon following the intake of the first dose (line 102), leading to a particularly high total concentration (line 106) at around the time the second dose is taken. In an extreme case, this could result in the subject experiencing an overdose, which could lead to medical complications. The graph shown in Fig. 3 represents a scenario where the second dose (line 104) is taken too long after the intake of the first dose (line 102), such that the total concentration (line 106) falls nearly to zero (i.e. potentially an ineffective therapeutic level) shortly before the second dose is taken. Among other advantages, embodiments disclosed herein help to provide an indication of an appropriate time window for taking the second dose such that the total concentration of the substance in the subject's blood remains within acceptable, safe and effective boundaries, such as in the example shown in Fig. 1.

In the embodiments disclosed herein, the volume of a substance taken by or administered to a subject (e.g. a person) may be referred to as a dose a bolus. As used herein, the term "bolus" is intended to refer to a discrete amount of medication, drug, or other compound taken or administered within a specific time, in order to raise its concentration in blood to an effective level. It can be difficult to determine when the concentration of a particular substance has reduced sufficiently such that a further bolus of the substance can safely be taken. It is possible to measure an amount (e.g. a concentration) of the substance in the subject's blood by taking periodic blood draws. The drawing of blood is obtrusive and can be painful; therefore, an alternative approach involves measuring the concentration of the substance present in the subject's sweat. However, as noted above, there is a time lag between a particular concentration of a substance in a subject's blood and the corresponding concentration of the substance in the subject's sweat. Similarly, there is a time lag between the occurrence a particular characteristic of the concentration profile (e.g. a maximum concentration) of a substance in a subject's blood and the same characteristic of the concentration profile of the substance in a subject's sweat. This time lag is taken into account in the disclosed embodiments. The time lag/difference may depend on, among other things, the nature of the substance (e.g. the type of medicine), and the subject, and the time lag may be influenced by factors such as physiological parameters of the subject, the age of the subject, the gender of the subject, and hormone levels in the subject, and interactions of the substance with other substances, such as food, drink and other medications. The subject's sweat rate and the location of the subject may also influence the time lag.

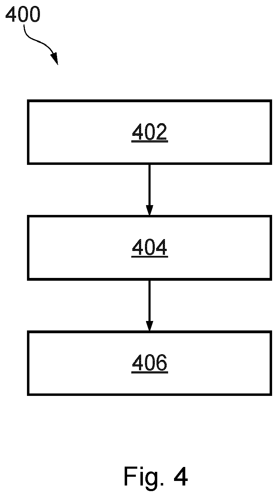

A first aspect of the present invention relates to a method. Fig. 4 is a flowchart of an example of a method 400. The method 400 may comprise a method, for example a method for determining the time window regarding the intake of a substance by a subject. The method 400 comprises, at step 402, receiving, from a sensor, first data indicative of a concentration of a first substance in sweat secreted from sweat glands of a subject. The sensor may, in some embodiments, form part of (e.g. be incorporated into or associated with) a sweat sensing device or sweat measurement device which may, in some examples, comprise a patch or device configured to be positioned on (e.g. attached to) the skin of the subject. In other embodiments, the sensor may comprise or form part of (e.g. be incorporated into or associated with) a wearable device such as a smart watch.

In this disclosure, the term "subject" is intended to include any living being, including a human or animal, capable of producing sweat. In some specific embodiments, the subject may comprise a human (i.e. a person) and may, for example, comprise a person undergoing medical treatment or a health assessment, which involves detecting and/or measuring a concentration of a substance in the sweat of the person. The first data indicative of a concentration of the first substance may, for example, comprise a measurement of the concentration of the first substance in the subject's sweat. In some embodiments, sweat secreted from sweat glands of the subject may be collected by a sweat collector (e.g. a sweat collection portion of a patch position on the subject's skin) and transported to the sensor for analysis. In other embodiments, secreted sweat may be received directly by the sensor and analyzed.

As noted above, the substance (e. g. the first substance) measured or analyzed by the sensor may comprise medication (e.g. a drug), a component of food or drink, or a biomarker present in the sweat and may be indicative of the presence of some other substance or a condition. Thus, in some embodiments, the sensor may comprise a biosensor. More generally, the substance may comprise a xenobiotic, and the sensor may comprise a biosensor for measuring a property (e.g. concentration) of the xenobiotic in sweat. A xenobiotic substance is one that is not naturally found or naturally produced within an organism (e. g. within the human body). The sensor may be configured or adapted to detect a particular substance for analysis or for measuring its concentration while, in other embodiments, the sensor may be configured to identify several substances and analyze or measure the concentration of one or more of them in sweat.

In some embodiments, the first data may comprise a single measurement of a concentration of the first substance in sweat (i.e. a measurement of the concentration of the first substance at a particular time). In other embodiments, however, the first data may comprise a plurality of measurements of the concentration of the first substance in sweat over a period of time. For example, measurements may be taken at intervals or periodically (e.g. every 30 seconds, every 1 minute, every 2 minutes, every 5 minutes, every 10 minutes, every 30 minutes, every 1 hour, or the like).

At step 404, the method 400 comprises determining a relationship between (i) a time of occurrence of a characteristic event in relation to the concentration of the first substance in sweat and (ii) a time of occurrence of the characteristic event in relation to the concentration of the first substance in blood. This step 404 may be carried out once and then used multiple times. For example, the results of step 404 may be stored in a look-up table.

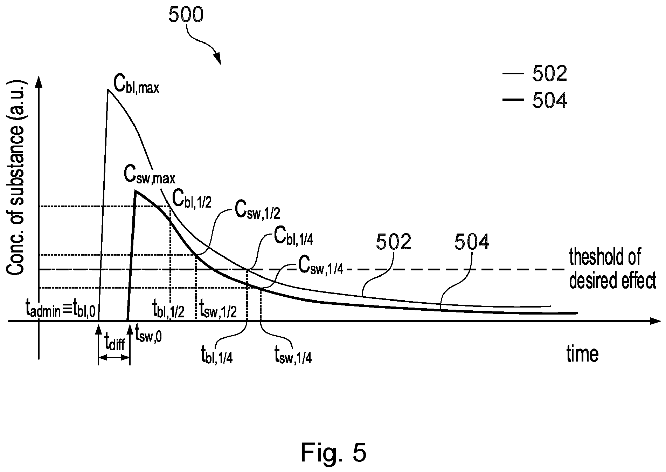

The method 400 comprises, at step 406, determining, based on the relationship and the received first data, a time window regarding the intake of a second substance by the subject. The time window may comprise one or more of a time window for the appropriate/safe intake of a substance or a time window during which intake of the substance could be unsafe/inappropriate. In some embodiments, the first substance and the second substance may comprise the same substance while, in other embodiments, the first substance and the second substance may comprise different substances. The first substance and/or the second substance may comprise a xenobiotic substance. Steps 404 and 406 are discussed in greater detail below, with reference to Fig. 5.

Fig. 5 is a graph 500 showing an example of a concentration of a substance (e.g. the first substance) in blood (line 502) and the concentration of the substance in sweat (504) as a function of time. Referring first to the line 502, the substance is administered to the subject (e.g. orally or intravenously) at $t_{admin}$. In this example, it is assumed that the substance is detectable in the subject's blood (i.e. the concentration of the substances measurable in the subject's blood) immediately after it is administered such that the time at which the first concentration change of the substance in blood, $t_{b1,0}$ is equivalent to the time at which the substance is administered (i.e. $t_{admin} = t_{b1,0}$). Thus, the substance starts to appear in the subject's blood at time $t_{b1,0}$, and the concentration rises sharply to a maximum concentration, $C_{b1,max}$. This represents a scenario where there is a very fast uptake of the substance into the subject's blood stream. The concentration of the substance in the subject's blood then starts to decrease in an approximately exponential manner. For the purposes of this disclosure, the decrease in the concentration of the substance in the subject's body (e.g. in sweat and in blood) is assumed to be exponential. According to the exponential model, the concentration of the substance in the subject's blood reduces by half after the expiry of the substance's half-life. In Fig. 5, the first half life occurs at a time, $t_{b1,1/2}$, and the concentration of the substance in the subject's blood at this time is given by $C_{b1,1/2}$. A second half-life occurs at time, $t_{b1,1/4}$, and the concentration of the substance in the subject's blood at this time is given by $C_{b1,1/4}$.

Referring now to the line 504, the time at which the first concentration change of the substance appears in sweat is indicated by $t_{sw,0}$, which occurs later than the appearance in the subject's blood, $t_{b1,0}$, by a time difference, $t_{diff}$. Again, the concentration of the substance in the subject's sweat rises sharply (due to the very fast uptake of the substance) to a maximum concentration, $C_{sw,max}$. The concentration of the substance in the subject's sweat decreases approximately exponentially, such that the concentration of the substance in the subject's sweat after a first half life, $t_{sw,1/2}$, is indicated by $C_{sw,1/2}$, and the concentration of the substance in the subject's sweat after a second half life, $t_{sw,1/4}$, is indicated by $C_{sw,1/4}$.

The "characteristic event" in relation to the concentration of the first substance, referred to in step 404, may comprise any event occurring in relation to the concentration of the substance as shown in the example of Fig. 5. For example, a characteristic event may be considered to occur at the times at which the concentration of the substance first begins to increase following its administration (i.e. $t_{b1,0}$ and $t_{sw,0}$). Another characteristic event may be considered to occur at the times at which the concentration of the substance is at a maximum (i.e. $C_{b1,max}$ and $C_{sw,max}$). Another characteristic event may be considered to occur when the concentration of the substance has reduced by half (e.g. at substance's half-life) (i.e. $C_{b1,1/2}$ and $C_{sw,1/2}$). Another characteristic event may be considered to occur, based on an indication from the subject, at the instant that an effect of the substance in the subject's body is first detected. Another characteristic event may be based on an estimate of the time taken for the substance to be detected in the subject's blood, the estimate being based on estimates or measurements in respect of a population of people (e.g. a population of people sharing similar physiological data, or from a similar demographic). Other characteristic events relating to the concentration of the first substance may also be used, and further examples are discussed below. In general, determining the relationship (step 404) may be based on at least one of: a determination of the time taken for the first substance to be detected in blood of the subject following intake of the first substance by the subject; an estimate of the time taken for the first substance to be detected in blood of the subject following intake of the first substance the subject, the estimate based on a population of people; an indication of the time taken for an effect of the first substance to be detected by a user (e.g. by the subject, a caregiver, an observer) following intake of the first substance by the subject; and an indication of the time taken for an effect of the first substance to be detected by a sensor following intake of the first substance by the subject. For example, as explained below, a sensor (e.g. an accelerometer) may be used to detect movement such as vibrations or shaking which are known effects caused by some medical conditions. Detection by the sensor that the effect has reduced may be indicative that the medication (i.e. the first substance) has started to take effect.

In the following example, $t_{diff}$ (i.e. the time delay of concentration changes (e.g. characteristic events) in sweat compared to equivalent changes in blood) may be approximated by the time difference or time delay between the time at which the substance is first administered, $t_{admin}$, and the time at which the first concentration change of the substance in sweat, $t_{sw,0}$, is measured. As noted above, in other examples, $t_{diff}$ may be represented by the time difference between the maximum concentrations in the blood and sweat or between other characteristic events occurring in the concentration v. time curves. It is assumed, in this example, that there is very little, or no, amount of the substance present in the subject's blood and sweat prior to the first administration of the substance.

The time difference, $t_{diff}$ in this example may be expressed as follows:

$$t_{diff} = t_{sw,0} - t_{admin} \qquad\qquad [1]$$

This situation applies for substances which have a negligible or very rapid uptake time, $t_{uptake}$, into the blood-stream of the subject, such that $t_{diff} \approx t_{diff} + t_{uptake}$ in the limit that $t_{uptake} \ll t_{diff}$. As noted above, this rapid uptake implies that $t_{admin} = t_{bl,0}$ (i.e. the time between the administration of the substance and its appearance in blood is negligible). Typically, rapid uptake occurs for substances which are administered by inhalation or intravenous injection. A value for $t_{admin}$ in equation [1] may be retrieved by a processor of a computing device performing the method 400 from, for example, a record associated with the subject, such as an electronic medical record (EMR), by manual user input into a user interface associated with the processor or, in an example in which the substance may be administered automatically, from an internal clock of the system or device administering the substance. Thus, according to a basic example, $t_{diff}$ may be determined by measuring the time between administering the substance (e.g. medication) to the subject and the substance appearing (e.g. an increase in the concentration of the substance from zero) in the subject's sweat.

Once $t_{diff}$ has been determined, the concentration of the substance in sweat can be directly mapped (e.g. by the processor) to the concentration in blood. An assumption made here is that a peak concentration of substance in blood, $C_{bl,max}$, will correspond directly to a peak concentration in sweat, $C_{sw,max}$, after the time difference, $t_{diff}$. A further assumption is that changes in the concentration of the substance in sweat, after correction for the time delay, correspond directly to changes in the concentration of the substance in blood (i.e. that the concentration trends in blood and sweat have a 1:1 correlation). However, the concentrations of the substance in blood and sweat may not have the same scalar magnitude, as the concentration of the substance in sweat may be lower or higher than the concentration of the substance in blood. In the example shown in Fig. 5, the maximum concentration of the substance in sweat is lower than the maximum concentration of the substance in blood.

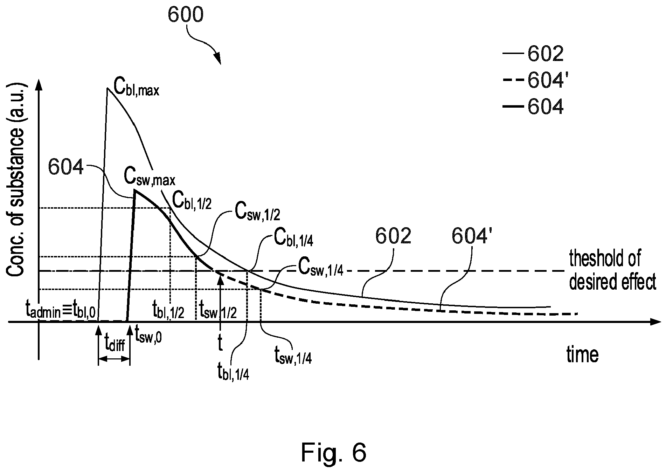

In practice, the relative concentration of the substance in blood at a given time, t, (i.e. $C_{bl}(t)/C_{bl,max}$) can be predicted by monitoring the relative concentration in sweat, $C_{sw}(t)/C_{sw,max}$, and through extrapolation using, for example, an exponential function fitting previously-acquired measurements, as shown in Fig. 6 and discussed in greater detail below. Alternatively, the relative concentration of the substance in blood at a given time, t, can be predicted using knowledge of the half-life, $t_{1/2}$, of the substance. For example, hydroxychloroquine is an example of a substance that may be detected in blood and sweat of a subject, and whose concentration may be measured. The half-life (i.e. the absorption half-life) of hydroxychloroquine is 3.6 hours. The relative concentration of the substance at a given time, t, in the subject's blood can be expressed as:

$$C_{bl}(t)/C_{bl,max} = C_{sw}(t+t_{diff})/C_{sw,max} \qquad [2]$$

where $C_{sw}(t)$ is given by:

$$C_{sw}(t) = C_{sw,max}*\exp(-\lambda(t-t_{diff})) \qquad [3]$$

where $\lambda$ is a positive number referred to as the decay constant of the decaying quantity (i.e. the substance), which is determined by the half-life of the substance (such that $\lambda = \ln(2)/t_{1/2}$).

It is noted that the time, t [hours], may be defined relative to the global time scale which is based on the initial administration of the active substance at time t = 0.

Fig. 6 is a graph 600 showing a further example of a concentration of a substance (e.g. the first substance) in blood (line 602) and the concentration of the substance in sweat (solid line 604) as a function of time. In this example, measurements of the concentration of the substance in the subject's sweat (solid line 604) are available only up to a particular time, and a dashed line 604' represents an estimation or prediction of the change in the concentration of the substance in sweat by extrapolating the concentration after the particular time.

To illustrate the use of such extrapolation to predict the concentration, an example is considered in which a substance with a half-life of 1 hour (i.e. $\lambda = \ln (2)/1 \approx 0.693$) is administered at time t = 0, and it takes 15 minutes for the substance to first appear in sweat, such that $t_{diff}$ = 0.25 hours. After 2 hours it is desired to know the relative concentration of the substance in the subject's blood, $C_{bl}(2)/C_{bl,max}$. Substituting all constants and values into Equation [2] and Equation [3], gives:

$$C_{sw}(t)/C_{sw,max} = \exp(-\ln(2)(2-0.25))= 0.297 \qquad [4]$$

which is the measured relative concentration of the substance in sweat, and:

$$C_{bl}(t)/C_{bl,max} = \exp(-\ln(2)(2)) = 0.250 \qquad [5]$$

which is the actual relative concentration of the substance in blood.

From this example, it is clear that, from the measured concentration at a given time, t, in sweat, $C_{sw}(t)$, the maximum concentration in sweat, $C_{sw,max}$, and the time difference, $t_{diff}$, it is possible to calculate the half-life and, subsequently, the relative concentration of the substance in blood can be determined as function of time.

It is to be noted that that, due to the time lag (i.e. $t_{diff}$), the relative concentration of the substance measured in sweat is much greater than the relative concentration of the substance measured in blood at a given time, since it takes time to for the substance to diffuse from the blood into the sweat glands via the interstitium.

Determining $t_{diff}$ may be considered to be a calibration step which is preferably performed when the substance is first administered to the subject. Once a modification for the time delay between the substance concentration in sweat and blood is made, then the relative concentration of the substance in blood may be determined at any point in time as described above. This enables several important parameters to be determined which are relevant to the safe or appropriate administration of the substance within an appropriate time window and/or the prevention of the unsafe or inappropriate administration of the substance within a time window.

In order to determine an appropriate time window within which a further amount of the substance may be administered to the subject, a latest administration time and an earliest administration time may be determined. The determination of the latest time is based on the intention that the concentration of the substance in the subject's body does not drop below a lower limit, and the determination of the earliest time is based on the intention that the concentration of the substance in the subject's body does not increase above an upper limit.

A lower therapeutic limit or threshold of desired effect of the substance in blood may be defined at time $t_{bl,ll}$ (where the subscript 'll' stands for lower limit) which occurs at a time earlier than when the corresponding lower limit is reached in sweat, $t_{sw,ll}$. The time difference or time delay between events relating to the concentration of the substance in sweat and in blood may also be used to determine $t_{bl,ll}$ using the expression:

$$t_{bl,ll} = t_{sw,ll} - t_{diff} \qquad [6]$$

In order to prevent the concentration of the substance in blood from reducing below the lower therapeutic limit,

$C_{bl,ll}$, a further administration of the substance (e.g. a further bolus or dose of medication) should be provided to the subject at the latest by a time, $t_{latest} = t_{bl,ll}$. This again assumes that the uptake of the substance is immediate.

For a given substance, $t_{latest}$ may be determined by rearranging and simplifying Equation [3] to solve for t, as follows:

$$t_{sw,ll} = -\ln(C_{sw,ll}/C_{sw,max})/\lambda + t_{diff} \qquad [7]$$

Therefore:

$$t_{latest} = t_{bl,ll} = t_{sw,ll} - t_{diff} = -\ln(C_{bl,ll}/C_{bl,max})/\lambda \qquad [8]$$

where $C_{sw,ll}$ is the lower therapeutic limit of the substance in sweat. When substituting Equation [6] into Equation [2], it can be derived that $C_{sw,ll}/C_{sw,max} \equiv Cbl_{ll}/C_{bl,max}$.

Equation [7] and Equation [8] imply that, due to the time lag, the lower limit of the concentration of the substance in blood will occur at a time when the relative concentration of substance in sweat will be higher than or equal to the relative concentration of the substance in blood.

As an example, a substance having a lower therapeutic limit of $C_{bl,max}/4$ and a half-life of 1 hour (i.e. $\lambda = \ln(2)/1 \approx 0.693$) is administered at time t = 0, and it takes 15 minutes to first appear in sweat, such that $t_{diff} = 0.25$ hours. It is possible to determine the latest time at which to administer a further amount of the substance (e.g. a bolus or dose of medication) by substituting the constants and values into Equation [8]:

$$t_{latest} = -\ln(0.25)/0.693 = 2 \text{ hours \{relative to } t_{admin}\} \qquad [9]$$

As noted above, determining the time window within which to administer a further amount of the substance may also involve determining the earliest time, $t_{earliest}$, when a further amount of the substance should be administered in order to prevent the concentration of the substance from exceeding an upper threshold limit, beyond which the subject could experience an overdose. This may occur, for example, if a second amount of the substance (e.g. a second bolus of medication) is administered too soon after the first amount of the substance (e.g. a first bolus of medication). The earliest time may be defined at a time, $t_{bl,ul}$ (where the subscript 'ul' stands for upper limit), which corresponds to the upper safe therapeutic limit of the substance in blood. The time difference or time delay between events relating to the concentration of the substance in sweat and in blood may also be used to determine $t_{bl,ul}$ using the expression:

$$t_{bl,ul} = t_{sw,ul} - t_{diff} \qquad [10]$$

In order to prevent the concentration of the substance in blood from increasing to or exceeding the upper therapeutic limit (i.e. a threshold of toxicity), $C_{bl,ul}$, a further administration of the substance (e.g. a further bolus or dose of medication) should be provided to the subject at the earliest by a time $t_{earliest} = t_{bl,ul}$.

For a given substance, $t_{earliest}$ may be determined by rearranging Equation [3] to solve for t, and recognizing that the amount of excess substance is given by $C_{bl,ul} - C_{bl,max}$, as follows:

$$t_{sw,ul} = -\ln((C_{sw,ul} - C_{sw,max})/C_{sw,max})/\lambda + t_{diff} \qquad [11]$$

Therefore:

$$t_{earliest} = t_{bl,ul} = t_{sw,ul} - t_{diff} = -\ln((C_{bl,ul} - C_{bl,max})/C_{bl,max})/\lambda \qquad [12]$$

where $C_{sw,ul}$ is the upper therapeutic limit of the substance in sweat.

As an example, a substance having an upper therapeutic limit of $3/2 * C_{bl,max}$ and a half-life of 1 hour (i.e. $\lambda = \ln(2)/1 \approx 0.693$) is administered at time t = 0, and it takes 15 minutes to first appear in sweat, such that $t_{diff} = 0.25$ hours. It is possible to determine the earliest time at which to administer a further amount of substance (e.g. a bolus of medication) by substituting the constants and values into Equation [12]:

$$t_{earliest} = t_{bl,ul} = t_{sw,ul} - t_{diff}$$

$$= -\ln((1.5-1)/1)/0.693 = 1 \text{ hour \{relative to } t_{admin}\}} \qquad [13]$$

Thus, an appropriate time window, $t_{app}$, for the administration of a further amount of the substance (e.g. a further bolus or dose of medication) may be defined based on $t_{earliest}$ and $t_{latest}$ as:

$$t_{earliest} \leq t_{app} \leq t_{latest} \qquad [14]$$

Thus, based on the above examples, 1 hour $\leq t_{app} \leq$ 2 hours.

Determining such an appropriate time window can be of particular importance and relevance to medications with narrow therapeutic windows, such as lithium.

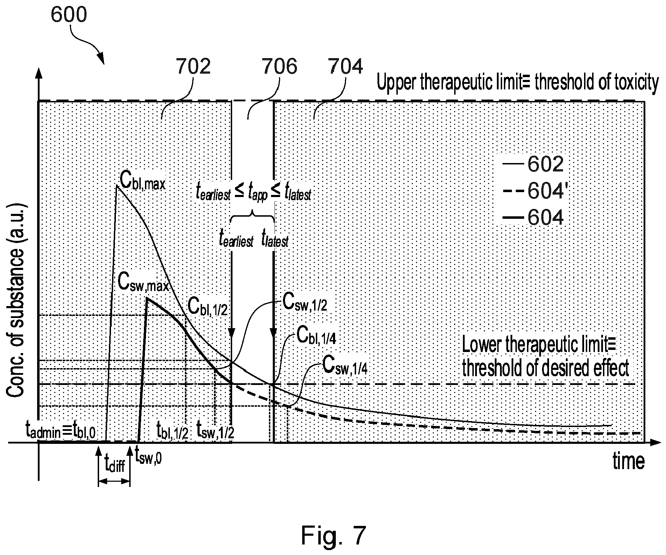

Fig. 7 shows the graph 600 of Fig. 6 with an indication of various time windows indicated. A first region 702 represents a period of time during which administration of a further amount of the substance would result in the concentration of the substance in the subject's blood exceeding the upper therapeutic limit. Thus, the first region 702 represents a time window that is too early for the administration of the further amount of substance, as it may result in an overdose. A second region 704 represents a period of time during which administration of a further amount of the substance would result in the concentration of the substance in the subject's blood reducing to below the lower therapeutic limit. Thus, the second region 704 represents a time window that is too late for the administration of the further amount of substance. A third region 706 represents a period of time during which the administration of the further amount of the substance is appropriate/safe. Thus, the third region 706 represents the time window $t_{app}$.

Thus, by measuring or determining values for $C_{sw}(t)$, $C_{sw,max}$ and $t_{diff}$, it is possible to derive the half-life and the earliest and latest times, $t_{earliest}$ and $t_{latest}$. By determining the earliest and latest times and, therefore, $t_{app}$, further actions may be taken aimed at ensuring or encouraging the administration of the substance at an appropriate time (e.g. within the determined time window) and prevention of the administration of the substance within an undesired time window (e.g. too early or too late). Such actions are discussed in greater detail below.

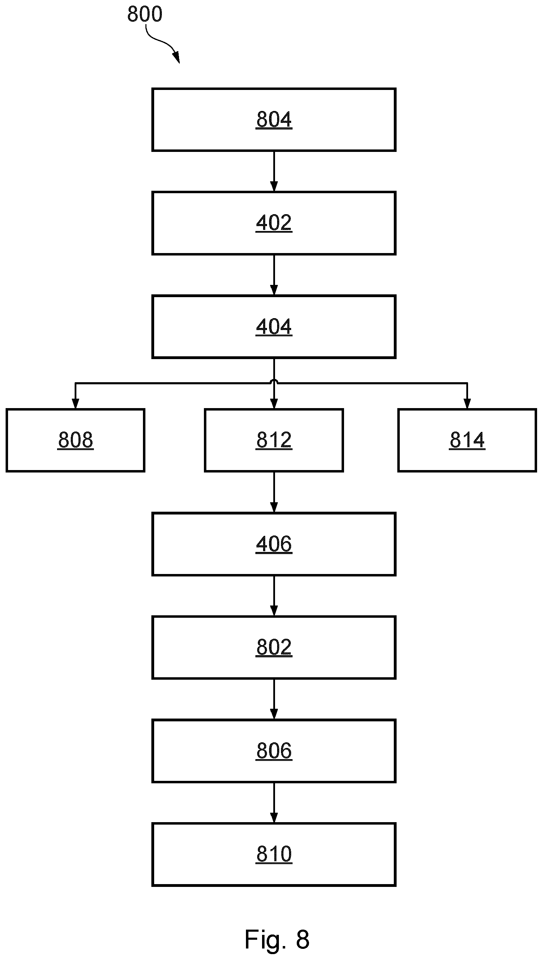

Fig. 8 is a flowchart of a further example of a method 800, which may also comprise a method, such as a method for determining the time window regarding the intake of a substance by a subject. The method 800 may comprise steps of the method 400 discussed above. At step 802, the method 800 may comprise determining, based on the relationship and the received first data, a concentration of the first substance in blood of the subject. This may be achieved, for example, if a relationship between the concentration of the substance in blood and sweat is known, e.g. for ammonium, the concentration in sweat is 20-50 times higher than the concentration in blood. Similarly, the concentration of the first substance in blood may be determined if a calibration has been performed; for example, if, when blood measurements are available, not only is the delay time, $t_{diff}$, determined, but also the relationship between the measured concentration in sweat and the measured concentration in blood is determined (taking into account the time lag, e.g. by looking at the peak concentrations).

As noted above, a measurement may be taken of the concentration of the first substance in the subject's blood when the substance is first administered to the subject. For example, in the case of a patient being administered medication, a blood sample or blood samples may be taken immediately or soon after the medication is first administered to the patient, so that an indication of the concentration of the medication in the subject's blood can be determined. From these measurements, it may be possible to determine, amongst other things, the time (after administration) at which the medication first appears in the patient's blood, the time at which the concentration of the medication in the patient's blood is at a maximum, and the actual maximum concentration of the medication in the patient's blood. Thus, at step 804, the method 800 may comprise, receiving, prior to receiving the first data, second data indicative of the time of occurrence of the characteristic event in relation to the concentration of the first substance in blood.

The determined time window may be used in several ways. In some embodiments, the time window during which the substance may be safely administered to the subject may be indicated to a user. Thus, the method 800 may comprise, at step 806, generating a signal to alert a user about the determined time window. The user may comprise the subject, a medical professional (e.g. a nurse administering medication to the patient) or any other person (e.g. a caregiver) for whom an alert about the determined time window would be of use. The signal generated at step 806 (e.g. by the processor performing the method 800) may comprise a signal configured to cause a device to provide the alert to the user. For example, the generated signal may cause a device to sound an alarm or provide a warning to the user that now is an appropriate time to administer a further amount of the substance. In some embodiments, an alert may be provided to the user via a user interface, visually, audibly, via haptic means or in some other manner. The user interface may comprise a user interface of a patient device or a device accessible to a patient and/or to a medical professional, such as a smart phone, a workstation, a wearable device (e.g. a smartwatch) or the like. For example, an alert may serve as a reminder to the user that a new bolus of medication should be administered to a patient by presenting a message to the user: "Please take a new bolus of medication in 30 to 45 minutes from now". Similarly, a warning alert may be provided at the time that the new bolus of medication should actually be administered: "Please take your medication now". If the user adheres to the alert and administers the substance/medication, the user may silence any further alerts. In some embodiments, alerts may be silenced automatically upon detection that a concentration of the substance in the sweat has begun to increase, or has increased above a threshold amount. If, however, the user does not administer medication, a further signal may be generated to alert the user, for example to urge the user to administer the substance/medication immediately: "The medication has not been administered during the appropriate time window. Please administer the medication as soon as possible".

In some embodiments, in addition to generating a signal to alert the user about the determined time window, the method 800 may further generate a signal to alert a user about the nature of the substance to be taken (e.g. which medication should be taken) and/or an amount of the substance that is to be taken (e.g. a dose of medication to be taken): e.g. "Please take [1 pill / 5 ml / 60 mg] of medication".

At step 808, the method 800 may further comprise obtaining physiological data relating to the subject. Such physiological data may comprise details of the subject, such as the subject's gender, age, medical background, weight or blood pressure, or other clinical data associated with the subject. Determining the time window (step 406) may be further based on the physiological data.

In one example, the physiological data may comprise data indicative of a time at which the subject starts to get tired, the time at which the subject typically goes to sleep and/or the time at which the subject typically wakes up. Such data may be taken into account when determining the time window, for example to take account of the subject's sleep-wake pattern. For example, the subject might have indicated that they typically wake up at 7 AM and could take medication soon after they wake up. In such an example, the user may be alerted or notified of one or more possibilities for taking the medication: "If you take your medication at 10 PM, you should take 60 mg. Alternatively, if you take your medication at 11 PM you should take 50 mg." The subject (e.g. a patient) is then able to decide what dose to take, depending at what time they would like to go to bed. The method may further take account of expected concentration levels and may offer the subject range of times only for those substances/medications that have a half-life that can cover the night without an additional bolus prior to sleep increasing the concentration of the substance beyond a threshold (e.g. dangerous) limit, and such that the concentration of the substance will be sufficiently high when the subject wakes up. Otherwise, the subject may have to wake up in the night to administer a further dose. In such examples, the method may involve generating a signal to advise the subject of an appropriate time window during the night when the further dose should be administered.

In some examples, the method 800 may comprise providing for presentation to a user (e.g. to the subject and/or to a medical professional) a visual representation (e.g. a graph) of the measured concentration of the substrate over time and/or an indication of the current situation of the substrate in their sweat and/or blood. Such information may be provided on a device (e.g. a smart phone) or on the device (e.g. a patch) performing the measurements on the subject. The determined time window (e.g. from $t_{earliest}$ to $t_{latest}$) may be displayed to the user, indicating visually the time remaining for substance (e.g. medication) to be administered.

In some embodiments, the device (e.g. processor) configured to perform the methods 400, 800 may be in communication with a device capable of administering the substance to the subject. Thus, the method 800 may further comprise, at step 810, generating a control signal to control delivery of the second substance at a time based on the determined time window. Such automated medication administration may be performed to circumvent the user forgetting or being unable to do administer the substance/medication themselves, or to ease the burden of a medical professional. Automatic medication administration may be achieved using, for example, an intravenous line or infusion pump.

In some embodiments, one or more factors may affect the concentration of the substance in the subject's sweat such that the concentration does not reduce exponentially, as expected. Thus, according to some embodiments, the first data indicative of a concentration of the first substance in sweat may comprise multiple measurements of the concentration of the first substance. The multiple measurements may be used to determine the time difference (or time delay), $t_{diff}$. In some embodiments, a measurement or a series of measurements of the concentration of the substance in the subject's sweat may be taken after the administration of each amount of the substance (e.g. after each bolus of medication has been administered). Each measurement may be used to calculate a time difference, $t_{diff}$, and an average may be calculated and used in further calculations. The comparison of the value of $t_{diff}$ calculated at different times may be used to determine any differences, and reasons for any differences, between varying values of $t_{diff}$ at different times of day, and actions may be taken based on the comparison. For example, it may be determined that the subject should reduce a dose of medication administered during the mornings.

The taking of additional measurements may be prompted or triggered by various events. For example, additional measurements may be taken if it is determined that the sweat rate of the subject has changed significantly (e.g. if they start to sweat more heavily or less heavily), because $t_{diff}$ might depend on the sweat rate. Additional measurements may, in some examples, be taken if there is a significant change in subject's weight. In other examples, additional measurements may be taken if a large change in hormonal levels is detected (e.g. related to menstruation). In this case,

$t_{diff}$ may be determined from a measurement having a sweat rate comparable to the current (or previous) one. In this situation, since the substance is already present in the subject's bloodstream, $t_{diff}$ will be determined based on the time after a new bolus is administered when the concentration of the substance in blood is at a peak, $t_{bl,max}$, and the time when a maximum in the concentration of the active substance in sweat occurs, $t_{sw,max}$. This can be expressed as:

$$t_{diff} = t_{sw,max} - t_{bl,max} \qquad [15]$$

For the case in which the substance uptake time is negligible, $t_{bl,max}$ will be equivalent to $t_{admin}$, as in the example shown in Fig. 5.

In the examples discussed above, it is assumed that the substrate uptake time in blood, following its administration to the subject, is very quick (i.e. negligible). However, in some cases, a substance may not have a rapid uptake into the bloodstream (i.e. $t_{uptake} \geq t_{diff}$ or $t_{uptake} < t_{diff}$ but not $<< t_{diff}$). This means that the time of administration of the substance, $t_{admin}$, is not indicative of the presence of the substance in the bloodstream and thus Equation [1] does not hold. This may occur for substances which are administered by ingestion or absorbed through the skin. In these cases:

$$t_{diff} = t_{sw,0} - t_{uptake} \qquad [16]$$

where, $t_{sw,0}$ is the time at which the first concentration change of the substance in sweat occurs (e.g. the first detectable change in concentration of the substance), and $t_{uptake}$ is given by:

$$t_{uptake} = t_{bl,0} - t_{admin} \qquad [17]$$

where $t_{bl,0}$ is the time at which the substance is first taken up in the blood (e.g. first detected in the blood) and $t_{admin}$ is the time at which the substance is first administered.

In practice, $t_{uptake}$ may be determined in one of several ways. For example, $t_{uptake}$ by measuring the concentration of the substance in a blood sample drawn from the patient at given moment in time after the first administration of the substance (i.e. an administration of the substance at a time when none of the substances currently in the subject's blood). This scenario may occur if a subject (e.g. patient) is in hospital when they first receive the substance and a blood gas analyzer is used for analysis of blood samples drawn from an arterial or intravenous line, for example using a blood gas analyzer present at the bedside in an intensive care unit (ICU) or in a central lab. Alternatively, as noted previously, a population-based estimate of the uptake time for a given substance may be used. To increase accuracy, the population-based estimate may be based on a particular population (e.g. a particular patient group), using parameters such as gender, age and especially body weight. In another example, the subject may provide a manual indication, via a user interface, of their complaints and/or when they feel the effect of the active substance is kicking in. As noted above, it is assumed that there is no existing concentration of the substance in the bloodstream of the subject which may confound the determination of $t_{bl,0}$.

In some embodiments, it may be possible to determine when a substance (i.e. a medication) is starting to take effect in the subject's body using another sensor. This may be linked to the rise of the concentration of the substance in the subject's blood. In example, body parts (e.g. a hand) of a subject suffering from Parkinson's disease may shake if medication has not been taken. Tremors/vibrations may be measured with a motion sensor such as an accelerometer, which could be worn for example as a bracelet or ring, or which could be measured every time when the subject uses their phone. A reduction in the shaking, as measured by the sensor, may be indicative that the medication has started to take effect in the subject's blood.

In an alternative embodiment, $t_{diff}$ may be determined based on the time difference between the time point at which the concentration of the substance in blood was measured, $t_{bl,meas}$, and the time point at which the relative concentration of the substance in sweat to the maximum concentration in sweat, matches the relative concentration of the substance in the measured blood sample (i.e. the time, t, where $C_{sw}/C_{sw,max} = C_{bl,meas}/C_{bl,max}$. It is possible to measure sufficient concentrations for this embodiment since measurements of the concentration of the substance in sweat can be taken semi-continuously (e.g. at regular intervals) without any particular discomfort for the subject. According to this embodiment, the time difference can be expressed as follows:

$$t_{diff} = t_{(C_{sw}/C_{sw,max} = C_{bl,meas}/C_{bl,max})} - t_{bl,meas} \qquad [18]$$

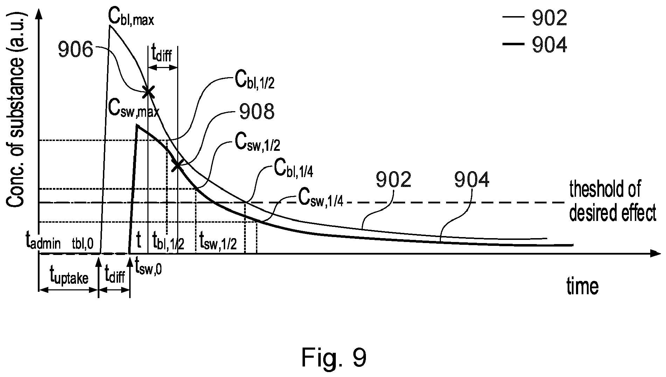

Fig. 9 is a graph 900 showing a further example of a concentration of a substance (e.g. the first substance) in blood (line 902) and the concentration of the substance in sweat (line 904) as a function of time, for a substance having a slow uptake into the blood (and sweat) of the subject, as discussed above. The time difference/delay between blood and sweat can be determined from a comparison of the characteristic events of the graph discussed above (e.g. when the concentrations first begin to increase, when the concentrations are at maximum or at the half lives of the concentrations). As discussed above, however, the time difference/delay in this example may be determined from the concentration of the sample measured in a blood sample drawn from the subject at an arbitrary time, t, (point 906 in Fig. 9) and a concentration measured in sweat at a time when the relative concentration in sweat matches the relative concentration of the substance in the blood sample at time t (point 908 in Fig. 9).

In this example, it is assumed that the measurement of the concentration of the substance in blood is carried out after the maximum concentration in blood is reached. This can be determined, for example by looking at the trend in the substance concentration in blood when two blood measurements are taken successively (e.g. by recognizing when the concentration starts decreasing) or by using timing based on a known pharmacological model, such as a pharmacokinetics-pharmacodynamics (PK-PD) model for a particular substance.

More generally, pharmacological models relating to any of the substances to be administered to the subject may be taken into account when determining the time window at step 406. The method 800 may, in some embodiments, comprise, at step 812, obtaining at least one of a first pharmacological model relating to the first substance and a second pharmacological model relating to the second substance. Thus, in the case that the first and second substances are the same substance, the same pharmacological model may be used while, in the case where the first and second substances are different, then separate pharmacological models may be obtained for use. Determining the time window (step 406) may further be based on at least one of the first pharmacological model and the second pharmacological model. As noted herein, one or more of the pharmacological models may be supplemented with physiological data relating to the subject.

In some embodiments, the substance administered to the subject may not itself be measurable in sweat. However, some substances may be metabolized or biotransformed by the subject's body to produce metabolites which can be detected in sweat and whose concentration in sweat can be measured. In such examples, the time of administration of the substance, $t_{admin}$, is not indicative of the presence of the metabolites in the subject's blood and is not indicative of the time taken for the metabolites to appear in the subject's sweat. In this example, therefore, Equation [1] no longer holds for determining $t_{diff}$. In addition to the uptake time, $t_{uptake}$, the metabolism or biotransformation time should be taken into account when determining $t_{diff}$:

$$t_{diff} = t_{sw,0} - (t_{uptake} + t_{metabolism}) \qquad [19]$$

where $t_{uptake}$ and $t_{metabolism}$ are given by:

$$t_{uptake} = t_{bl,0} - t_{admin} \qquad [20]$$

$$t_{metabolism} = t_{bl,metabolite} - t_{bl,0} \qquad [21]$$

where $t_{admin}$ is the time at which the substance is first administered to the subject, $t_{bl,0}$ is the time at which the substance first appears in blood and $t_{bl,metabolite}$ is time at which the metabolite of the substance is first present in blood.

Substituting Equation [20] and Equation [21] into Equation [19] leads to the following simplified expression for $t_{diff}$:

$$t_{diff} = t_{sw,0} - (t_{bl,metabolite} - t_{admin}) = t_{sw,0} - t_{bl,metabolite} \qquad [22]$$

$$[\text{since } t_{admin} \to 0]$$

It should be noted that Equation [22] holds for substances which have a rapid uptake time, $t_{uptake}$, into the bloodstream as well as those with a long uptake time. Moreover, it should be noted that metabolism of a substance is not instantaneous, therefore $t_{bl,metabolite} - t_{admin}$ will always be > 0.

In practice, $t_{bl,metabolite}$ may be determined in one of several ways. For example, $t_{bl,metabolite}$ may be determined using the concentration of the metabolite measured in a blood sample drawn from the patient at given moment in time after the first administration of the active substance. This scenario may occur if a subject is in hospital when they first receive the substance but, in examples where a blood draw is not possible, other methods, as discussed above, may be used.

In an alternative embodiment, $t_{diff}$ may be determined based on the time difference between the time point at which the concentration of the metabolite in blood was measured, $t_{bl,metabolite\_meas}$, and the time point at which the relative concentration of the substance in sweat to the maximum concentration in sweat, matches the relative concentration of the metabolite in the measured blood sample (i.e. the time, t, where $C_{sw,metabolite}/C_{sw,metabolite,max}$ = $C_{bl,metabolite\_meas}/C_{bl,metabolite,max}$. According to this embodiment, the time difference can be expressed as follows:

$$t_{diff} = t_{bl,metabolite\_meas} = t_{where}(C_{bl,metabolite\_meas}/C_{bl,metabolite,max}) - t_{bl,metabolite\_meas} \quad [23]$$

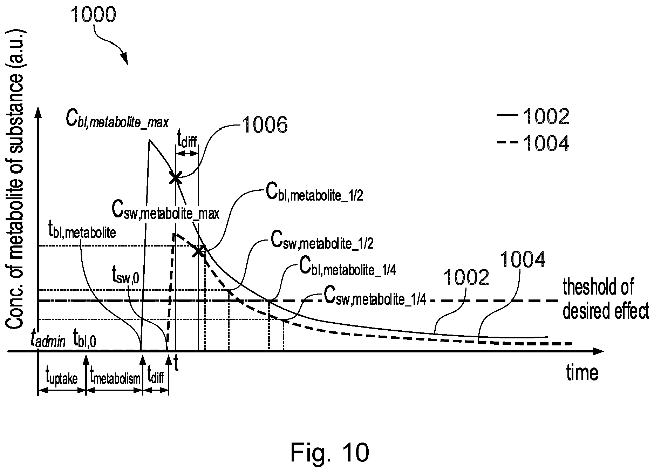

Fig. 10 is a graph 1000 showing an example of a concentration of a metabolite of a substance (e.g. a metabolite of the first substance) in blood (line 1002) and the concentration of the metabolite of substance in sweat (line 1004) as a function of time. Similar to the example shown in Fig. 9 above, the time difference/delay between blood and sweat can be determined not only from a comparison of the characteristic events of the graph discussed above (e.g. when the concentrations first begin to increase, when the concentrations are at maximum and at the half lives of the concentrations), but also from the concentration of the metabolite in the sample measured in a blood sample drawn from the subject at an arbitrary time, t, (point 1006 in Fig. 10) and a concentration of the metabolite measured in sweat at a time when the relative concentration of the metabolite in sweat matches the relative concentration of the metabolite in the blood sample at time t (point 1008 in Fig. 10).

Thus, from the above examples, it is clear that a measurement of the concentration of a metabolite of the first substance in sweat can also be used to determine the time difference, $t_{diff}$, and, therefore, a time window regarding the appropriate/safe administration of a further amount of the first substance. Thus, according to some examples, the received first data may comprise data indicative of a concentration of a metabolite of the first substance in the secreted sweat from sweat glands of a subject over a period of time. Determining the relationship (step 404), may comprise determining a relationship between the time of occurrence of a characteristic event in relation to the concentration of the metabolite of the first substance in sweat and the time of occurrence of the characteristic event in relation to the concentration of the metabolite of the first substance in blood.

It is noted in various examples discussed herein that a pharmacological model may be used to aid the determination of the relationship (step 404) and/or the time window (step 406). Since the metabolic rate of a substance varies from person to person, physiological data relating to the subject may also be used to aid these determinations. Thus, referring again to Fig. 8, the method 800 may comprise, at step 814, determining, using a pharmacological model relating to the first substance and physiological data relating to the subject, a metabolic rate of the first substance in the subject. Determining the time window (step 406) may be further based on the determined metabolic rate.

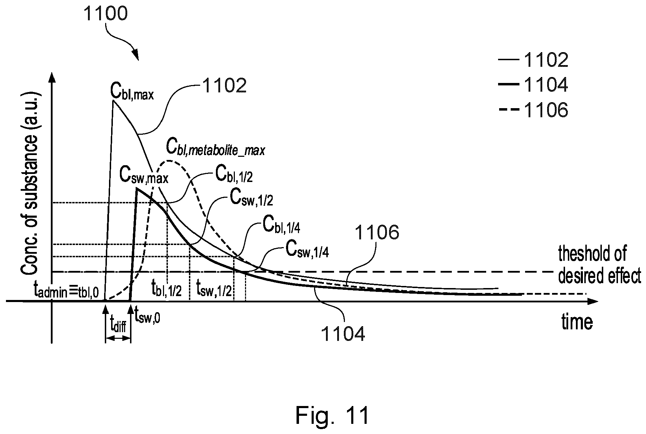

In some embodiments, a substance (e.g. the first substance) may be measurable in sweat, but the substance may be metabolized in the subject's body, and the metabolites of the substance may not be measurable in sweat. In this example, the metabolites are considered to be "active" metabolites as they cause an effect within the subject's body, while the (un-metabolized) substance is considered "inactive" as it does not cause an effect within the subject's body. In this example, the time difference, $t_{diff}$, may be determined using Equation [1] or Equation [16] or Equation [19], depending on whether the substance has negligible uptake or long uptake in the blood, or is metabolized or bio-transformed within the human body. However, as noted, the metabolite of the substance is active and the substance itself is not active. Therefore, in this embodiment the concentration of the inactive substance in sweat is used to determine the concentration of the inactive substance in blood. The concentration of the active metabolite in blood can therefore be inferred. It is noted that the concentration of the metabolite is influenced not just by the rate at which the inactive substance is metabolized or bio-transformed in the body, but also by the rate at which the active metabolite is consumed by the body. These two rates are typically non-equal, which leads to a non-trivial transfer function between the concentration of inactive substance and the concentration of active metabolite. This may be overcome by obtaining and using a pharmacological model (e.g. a pharmacokinetics-pharmacodynamics, or PK-PD model). Data indicating the rate of absorption and subsequent metabolism of medications after ingestion are generally available from pharmaceutical companies for each substance or drug, and are typically based on the subject's age, body weight and gender. With the input of these patient-specific data, the drug metabolite concentration may be estimated from metabolic data obtainable from a pharmaceutical database or from a pharmacological model. Once the metabolite concentration has been determined, then it can be correlated with the concentration of the inactive substance in sweat. An example of this embodiment is illustrated in Fig. 11 below.

Fig. 11 is a graph 1100 showing an example of a concentration of a substance (e.g. the first substance) in blood (line 1102) and the concentration of the substance in sweat (line 1104) as a function of time. In this example, a dashed line 1106 indicates the concentration of a metabolite of the substance, as inferred or predicted based on a pharmacological model using patient-specific data, and the concentration of the inactive substance in blood, determined using measurements of the concentration of the inactive substance in sweat, by taking account of the time difference, $t_{diff}$.

So far, embodiments of the invention have been discussed in which the second amount of substance to be administered is the same substance as delivered during the first administration. In other words, in the method 400, the first substance and the second substance are the same substance. However, different substances (e.g. active substances) may interact with one another. For example, medication or nutrition (e.g. vitamins, minerals or nutrition, such as a food or drink ingredient) may interact with other types of medication or nutrition. In some cases, interactions between different substances may lead to an adverse effect in the subject's body and, therefore, should be avoided or minimized. To achieve this, embodiments of the present disclosure enable the determination of a suitable time window within which the second substance can safely be administered following the administration of a first, different substance.

The steps of the method 400 may be performed in respect of each type of substance (e.g. each medication and ingredient to be administered to/consumed by a subject). The determination of the time difference, $t_{diff}$, and/or the time difference taking into account any uptake time, $t_{diff} + t_{uptake}$, may be carried out as a calibration step for each substance. For example, this may be done for at least one of: a medication, a nutrient, and an ingredient in the first substance and a medication, a nutrient and an ingredient with which it reacts. Once the time differences have been determined, time windows may be determined, and information or advice may be provided to a user (e.g. to the subject and/or to a medical professional) regarding when to and when not to administer medication or consume a substance. For example:

- for a subject who has taken medication that should not be combined with alcohol, a 'green light' (e.g. allowance notification) may be provided for presentation to the user when it is again safe for the subject to consume alcohol and/or a 'red light' (e.g. warning notification) may be provided for presentation to the user when alcohol should not be consumed;
- for a subject who is to take medication that can be taken after drinking alcohol, the subject may be notified when it is safe to do so. For example, by extrapolation, it can be determined the time at which the alcohol concentration in sweat will drop below a lower limit, and subtracting $t_{diff,alcohol} + t_{uptake,medication}$ from that time gives the earliest time at which the medication can be taken;
- a subject may be notified of a safe or good time window in which to take an iron supplement after vitamin C has been taken, or a safe or good time window in which to take vitamin C when an iron supplement has been taken (as vitamin C enhances the uptake of iron by the body); or
- a subject may be notified of time window in which calcium should not be taken after an iron supplement has been taken, or a time window in which an iron supplement should not be taken after calcium has been taken (as calcium reduces the uptake of iron).

In a further refinement of this embodiment it is possible to determine if potentiation or depotentiation has occurred (e.g. when the effect of a substance is enhanced in the presence of another substance or when one drug acts to reduce the effect of another drug). This might occur for instance if a patient eats a food such as grapefruit which is known to interact with some medications. In such examples, the method may comprise generating a signal for notifying or warning the subject that a food-drug interaction has occurred. This may be especially important in the case of potentiation since the effect of the medication on the patient may be stronger despite the administration of an appropriate bolus of medication.

Thus, some embodiments of the present disclosure may be used in scenarios in which the first substance and the second substance interact with one another, or wherein a metabolic product (e.g. metabolites) of the first substance and the second substance interact with one another.

In the examples discussed above, an interaction between various substances may be detected from the concentration of the substance measured in the sweat. For example, if a concentration of a particular substance (e.g. a biomarker) is significantly higher or lower than expected, then it may be determined that a particular drug is not acting as expected. Thus, it may be determined that some interaction has taken place. Similarly, if the reducing concentration deviates from the expected exponential decrease, then it may be determined that another substance is interacting with the substance to reduce (or enhance) its effect.

In some cases, a subject may be taking multiple different substances/medications at the same time, whereby each substance has a distinct uptake in blood and drug metabolism/biotransformation. For such cases, the first time each drug is administered, $t_{diff+tuptake}$ and/or $t_{diff}$, $t_{latest}$, and $t_{earliest}$, can be determined and recorded for each substance (e.g. drug A, drug B, drug C, etc. or metabolite A, metabolite B, metabolite C, etc.) using the methods described herein. It is noted that these different substances may be administered at different points in time (i.e. non-simultaneously). In some embodiments, therefore, the method may comprise generating a signal to provide an alert/a reminder/a warning/advice or the like to guide the patient and or/user when the different medications should and/or should not be administered. For example, the subject may be provided with a notification saying "Please take medication A at 12:00, medication B at 12:30 and medication C at 13:00".

Additionally, in some embodiments, the amounts or dose of each medication may be advised. For example, the subject may be provided with a notification saying "Please take 1 pill of medication A at 12:00, 10 mg of medication B at 12:30 and 60 ml of medication C at 13:00".

In a further embodiment, the method may detect, based on the concentration of the medication in the subject's sweat, if an incorrect medication or amount of medication was taken by the patient, or if the medication was taken at the incorrect time. A warning/alert/alarm can be provided to the subject and/or to a medical professional. This is particularly advantageous since many elderly polypharma patients have challenges in administering the right medication at the right time due to the large number of different medications they are required to administer.

In some examples described herein, the substance may be delivered or administered to the subject as a bolus. However, in some medication administration systems, such as those used with infusion therapy, medical drugs or nutrients may be dissolved in an infusion liquid for administration, and are not administered as a bolus, but are applied by regulating the flow rate of infusion liquid into the human body. Some of the above-described embodiments cannot, therefore, be used with infusion therapy systems. In such systems, a semi-bolus dosing scheme may be implemented. When the infusion therapy is started, the flow rate is set to a high value for a short time period and, subsequently, reduced to a normal flow rate. A bolus of substance is then administered (e.g. injected), and this enables a determination to be made of the concentration of the substance in blood from the measured concentrations in sweat. Parameters may be determined as described in the embodiments above, that allow for the determination of the concentration of the substance in the subject's blood from a measurement of the concentration of the substance in the subject sweat, even in the period where no bolus is given. In this way, a determination of the concentration of the substance in the subject's blood can be made based on measurements of the concentration of the substance in the subject's sweat during infusion therapy. As such, the determined information can be used to increase or decrease the flow rate of the infusion liquid. This may apply to drugs that are naturally occurring the human body, such as insulin.

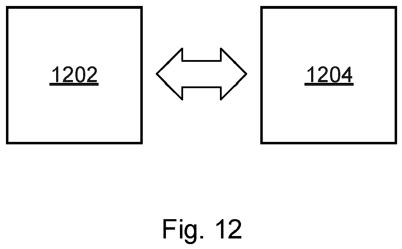

It will be clear from the above discussion that steps of the methods 400, 800 may be performed using one or more processors of one or more computing devices. Thus, a further aspect of the present invention provides a computer program product. Fig. 12 is a schematic illustration of an example of a processor 1202 in communication with a computer-readable medium 1204. According to some embodiments, a computer program product may comprise a non-transitory computer-readable medium 1204, the computer-readable medium having computer-readable code embodied therein, the computer-readable code being configured such that, on execution by a suitable computer or processor 1202, the computer or processor is caused to perform steps of the methods 400, 800 disclosed herein.

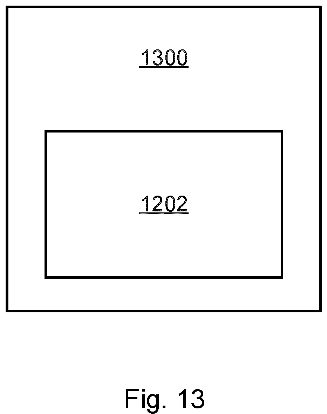

Similarly, the processor 1202 may form part of an apparatus, such as a computing device (e.g. a desktop computer, a laptop computer, a tablet computer, a smart phone, or a wearable device) or a server. Thus, a further aspect of the present invention provides an apparatus. Fig. 13 is a schematic illustration of an example of an apparatus 1300. The apparatus 1300 comprises a processor 1202 configured to perform steps of the methods 400, 800 disclosed herein. In some embodiments, the apparatus 1300 may further comprise the computer-readable medium 1204.

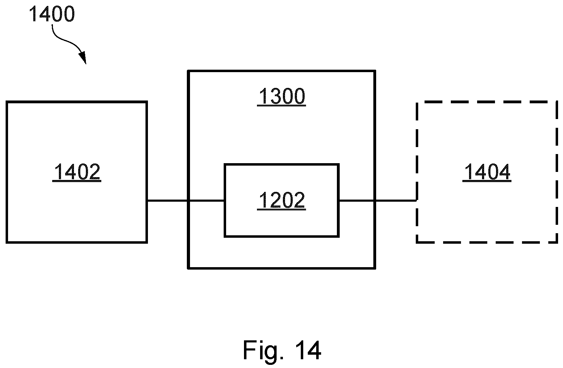

In some embodiments, the apparatus 1300 may further include a sensor for measuring, amongst other things, a concentration of a substance in the subject sweat. In other embodiments, however, the sensor and the apparatus 1300 may be separate components capable of communicating with one another as part of a system. Fig. 14 is a schematic illustration of an example of such a system 1400. The system 1400 comprises a sweat sensor 1402 for measuring data indicative of a characteristic of sweat generated by sweat glands of a subject. For example, the sweat sensor 1402 may comprise a sensor capable of measuring a concentration of a substance (e.g. the first substance, a biomarker, or the like) in the subject's sweat. The system 1400 further comprises an apparatus as disclosed herein (e.g. the apparatus 1300). The first data received by the processor 1202 of the apparatus 1300 is received from the sweat sensor 1402.

In some embodiments, the system 1400 may further comprise a user interface 1404 configured to provide a notification to the subject at a time based on the determined time window. For example, the user interface 1404 may comprise a display or a speaker (e.g. of a portable device such as a smart phone or of the apparatus 1300 itself), to provide a user (e.g. the subject or a medical professional) with information about the determined time window. For example, as noted above, such a notification may inform the user that it is safe to take or administer the second substance, or that it is currently unsafe to take or administer the second substance. Other alerts, notifications and warnings may be provided via the user interface 1404, as described herein.

The embodiments described herein provide a mechanism by which valuable information can be determined from measurements taken from a subject's sweat. Analyzing a subject sweat is far less intrusive than drawing blood from the subject for analysis. Based on the data acquired in respect of the sweat, a relationship between the concentration of a substance in the sweat and the concentration of the substance in the subject's blood can be determined, and it is then possible to determine a time window regarding the administration of a further substance to the subject. Thus, based on just measurements taken in respect of the subject's sweat, the subject can be informed of whether or not it is safe to take a further amount of the substance or a different substance, such as a different medication.

The processor 1202 can comprise one or more processors, processing units, multi-core processors or modules that are configured or programmed to control the apparatus 1300 in the manner described herein. In particular implementations, the processor 1202 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. Measures recited in mutually different dependent claims can be advantageously used in combination. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1.  A method (400) comprising using a processor (1202) for:

    receiving (402), from a sensor, first data indicative of a concentration of a first substance in sweat secreted from sweat glands of a subject; and
    determining (406) a time window regarding the intake of a second substance by the subject, based on the received first data and a relationship between one the one hand, a time of occurrence of a characteristic event in relation to the concentration of the first substance in sweat, and on the other hand, a time of occurrence of the characteristic event in relation to the concentration of the first substance in blood.

2.  A method (400, 800) according to claim 1, further comprising:
    determining (802), based on the relationship and the received first data, a concentration of the first substance in blood of the subject.

3.  A method (400, 800) according to claim 1 or claim 2, further comprising:
    receiving (804), prior to receiving the first data, second data indicative of the time of occurrence of the characteristic event in relation to the concentration of the first substance in blood.

4.  A method (400, 800) according to any of the preceding claims, further comprising:

generating (806) a signal to alert a user about the determined time window.

5. A method (400, 800) according to any of the preceding claims, further comprising:
generating (810) a control signal to control delivery of the second substance at a time based on the determined time window.

6. A method (400, 800) according to any of the preceding claims, further comprising:

obtaining (808) physiological data relating to the subject;
wherein determining the time window is further based on the physiological data.

7. A method (400, 800) according to any of the preceding claims, further comprising:

obtaining (812) at least one of a first pharmacological model relating to the first substance and a second pharmacological model relating to the second substance;
wherein determining the time window is further based on at least one of the first pharmacological model and the second pharmacological model.

8. A method (400, 800) according to any of the preceding claims, wherein the determining the relationship is based on at least one of: a determination of the time taken for the first substance to be detected in blood of the subject following intake of the first substance by the subject; an estimate of the time taken for the first substance to be detected in blood of the subject following intake of the first substance the subject, the estimate based on a population of people; an indication of the time taken for an effect of the first substance to be detected by a user following intake of the first substance by the subject; and an indication of the time taken for an effect of the first substance to be detected by a sensor following intake of the first substance by the subject.

9. A method (400, 800) according to any of the preceding claims, wherein the received first data comprises data indicative of a concentration of a metabolite of the first substance in the secreted sweat from sweat glands of a subject over a period of time; and
wherein the determining the relationship comprises determining a relationship between the time of occurrence of a characteristic event in relation to the concentration of the metabolite of the first substance in sweat and the time of occurrence of the characteristic event in relation to the concentration of the metabolite of the first substance in blood.

10. A method (400, 800) according to any of the preceding claims, further comprising:

determining (814), using a pharmacological model relating to the first substance and physiological data relating to the subject, a metabolic rate of the first substance in the subject;
wherein determining the time window is further based on the determined metabolic rate.

11. A method (400, 800) according to any of the preceding claims, wherein the first substance and the second substance interact with one another; or wherein a metabolic product of the first substance and the second substance interact with one another.

12. A computer program product comprising computer-readable code being configured such that, on execution by a suitable computer or processor (1202), the computer or processor is caused to perform the method of any of the preceding claims.

13. An apparatus (1300) comprising:
a processor (1202) configured to perform the steps of the method of any of claims 1 to 11.

14. A system (1400) comprising:

a sweat sensor (1402) for measuring data indicative of a characteristic of sweat generated by sweat glands of a subject; and
an apparatus (1300) according to claim 13;
wherein the first data is received from the sweat sensor.

15. A system (1400) according to claim 14, further comprising:

a user interface (1404) configured to provide a notification to the subject at a time based on the determined time window.

Fig. 1

Fig. 2

Fig. 3

400

402

404

406

Fig. 4

Fig. 5

Fig. 6

Fig. 7

800

Fig. 8

Fig. 9

Fig. 10

Fig. 11

Fig. 12

1300

1202

Fig. 13

1400

1300

1402

1202

1404

Fig. 14

Europäisches
Patentamt
European
Patent Office
Office européen
des brevets

# EUROPEAN SEARCH REPORT

Application Number

EP 21 15 1287

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/296823 A1 (MELKER RICHARD J [US] ET AL) 7 November 2013 (2013-11-07) * paragraphs [0021] - [0311] * | 1-15 | INV. G16H20/10 |
| X | MAGE P. L. ET AL: "Closed-loop control of circulating drug levels in live animals", NATURE BIOMEDICAL ENGINEERING, vol. 1, no. 5, 1 May 2017 (2017-05-01), XP055818259, DOI: 10.1038/s41551-017-0070 Retrieved from the Internet: URL:https://www.researchgate.net/profile/Peter-Mage/publication/316846247_Closed-loop_control_of_circulating_drug_levels_in_live_animals/links/5a71f3e4458515512075d5d8/Closed-loop-control-of-circulating-drug-levels-in-live-animals.pdf> * the whole document * | 1-15 | |
| X | Teymourian Hazhir ET AL: "Wearable Electrochemical Sensors for the Monitoring and Screening of Drugs", ACS sensors, 25 September 2020 (2020-09-25), pages 2679-2700, XP055817948, United States DOI: 10.1021/acssensors.0c01318 Retrieved from the Internet: URL:https://bordersens.eu/wp-content/uploads/2020/10/Wearable-Sensors-Drug-Analysis.pdf [retrieved on 2021-06-24] * the whole document * | 1-15 | TECHNICAL FIELDS SEARCHED (IPC) G16H |

-/--

The present search report has been drawn up for all claims

| Place of search | Date of completion of the search | Examiner |
|---|---|---|
| Munich | 30 June 2021 | Díaz de Lezana, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 21 15 1287

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Anonymous: "Guideline on the investigation of drug interactions", , 21 June 2012 (2012-06-21), pages 2-59, XP055727049, Internet Retrieved from the Internet: URL:https://www.ema.europa.eu/en/documents /scientific-guideline/guideline-investigat ion-drug-interactions-revision-1_en.pdf [retrieved on 2020-09-02] * the whole document * ----- | 1-15 | |
| A | BIAN SUMIN ET AL: "Towards wearable and implantable continuous drug monitoring: A review", JOURNAL OF PHARMACEUTICAL ANALYSIS, vol. 11, no. 1, 15 August 2020 (2020-08-15), pages 1-14, XP055817942, ISSN: 2095-1779, DOI: 10.1016/j.jpha.2020.08.001 Retrieved from the Internet: URL:https://www.ncbi.nlm.nih.gov/pmc/artic les/PMC7428759/pdf/main.pdf> * the whole document * ----- | 1-15 | TECHNICAL FIELDS SEARCHED (IPC) |

The present search report has been drawn up for all claims

| Place of search | Date of completion of the search | Examiner |
|---|---|---|
| Munich | 30 June 2021 | Díaz de Lezana, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 15 1287

This annex lists the patent family members relating to the patent documents cited in the above-mentioned European search report.
The members are as contained in the European Patent Office EDP file on
The European Patent Office is in no way liable for these particulars which are merely given for the purpose of information.

30-06-2021

| Patent document cited in search report | Publication date | Patent family member(s) | Publication date |
|---|---|---|---|
| US 2013296823 A1 | 07-11-2013 | CA 2808457 A1<br>EP 2605813 A2<br>US 2013296823 A1<br>WO 2012024401 A2 | 23-02-2012<br>26-06-2013<br>07-11-2013<br>23-02-2012 |

EPO FORM P0459

For more details about this annex : see Official Journal of the European Patent Office, No. 12/82